# EUROPEAN PATENT SPECIFICATION

(11) **EP 3 217 173 B1**
(45) Date of publication and mention of the grant of the patent: **01.01.2020**
(21) Application number: 15857668.6
(22) Date of filing: 30.10.2015
(51) Int. Cl.: G01N 33/50, G01N 27/62, G01N 33/92, C07H 15/10

(54) **METHOD FOR DETECTING DISEASE**
VERFAHREN FÜR DEN NACHWEIS EINER ERKRANKUNG
PROCÉDÉ DE DÉTECTION DE MALADIES

(30) Priority: 05.11.2014 JP 2014224811
(43) Date of publication of application: 13.09.2017
(73) Proprietor: The Noguchi Institute, Tokyo 1730003 (JP)
(72) Inventor: INOKUCHI, Jin-ichi, Sendai-shi Miyagi 981-3203 (JP); VEILLON, Lucas, Houston, TX 77035 (US)
(74) Representative: dompatent von Kreisler Selting Werner - Partnerschaft von Patent- und Rechtsanwälten mbB
(86) International application number: PCT/JP2015/080801
(87) International publication number: WO 2016/072364

(56) References cited:
- WO-A1-2005/067971
- WO-A1-2007/139224
- WO-A1-2007/139224
- WO-A1-2011/065389
- WO-A1-2012/122602
- JP-A- H0 193 562
- L Riboni ET AL: "Changes of the human liver GM3 ganglioside molecular species during aging", European journal of biochemistry / FEBS, 15 January 1992 (1992-01-15), pages 107-113, XP055464967, GERMANY DOI: 10.1111/j.1432-1033.1992.tb19834.x Retrieved from the Internet: URL:https://onlinelibrary.wiley.com/doi/pd f/10.1111/j.1432-1033.1992.tb19834.x
- Stephan Ladisch ET AL: "Immunosuppressive Activity of Chemically Synthesized Gangliosides1", Biochemistry, 1 January 1995 (1995-01-01), XP055465080, Retrieved from the Internet: URL:https://pubs.acs.org/doi/pdf/10.1021/b i00004a012
- MIKAMI M ET AL: "Menstrual cycle-associated expression of 2-hydroxy fatty acyl phytosphingosine-containing GlcCer, LacCer and Gb3Cer in human uterine endometrium", BIOCHIMICA ET BIOPHYSICA ACTA - LIPIDS AND LIPID METABOLISM, ELSEVIER SCIENCE BV. AMSTERDAM, NL, vol. 1125, no. 1, 8 April 1992 (1992-04-08), pages 104-109, XP023366491, ISSN: 0005-2760, DOI: 10.1016/0005-2760(92)90162-O [retrieved on 1992-04-08]
- SATO T ET AL: "Circulating levels of ganglioside GM3 in metabolic syndrome: A pilot study", OBESITY RESEARCH & CLINICAL PRACTICE, ELSEVIER, AMSTERDAM, NL, vol. 2, no. 4, 1 December 2008 (2008-12-01), pages 231-238, XP025659141, ISSN: 1871-403X, DOI: 10.1016/J.ORCP.2008.06.001 [retrieved on 2008-07-22]
- LUCAS VEILLON ET AL: "Identification of Ganglioside GM3 Molecular Species in Human Serum Associated with Risk Factors of Metabolic Syndrome", PLOS ONE, vol. 10, no. 6, 1 June 2015 (2015-06-01), page e0129645, XP055464817, DOI: 10.1371/journal.pone.0129645
- INOKUCHI J: 'GM3 and diabetes' GLYCOCONJ J vol. 31, no. 3, 01 January 2014, pages 193 - 197, XP055440408 DOI: 10.1007/S10719-013-9516-4
- HSU FF ET AL.: 'Electrospray ionization tandem mass spectrometric analysis of sulfatide. Determination of fragmentation patterns and characterization of molecular species expressed in brain and in pancreatic islets' BIOCHIM BIOPHYS ACTA vol. 1392, no. 2-3, 15 June 1998, pages 202 - 216, XP009018760

## Description

### Technical field

The present invention relates to a method for detecting specific diseases or detecting onset risk of these diseases and a detection kit therefor, more specifically to a method for detecting specific diseases or detecting onset risk of these diseases, comprising a step of measuring a ganglioside GM3 with a specific structure. **Also disclosed is a detection kit therefor.**

### BACKGROUND ART

Diabetes and cardiovascular disease (CVD) are related diseases that share a number of overlapping risk factors including obesity, hypertension and low levels of physical activity. Atherosclerosis which is one of the CVDs is a pathological process characterized by the accumulation of cholesterol and fatty material in the lumen of blood vessels resulting in plaque formation. Due to plaque rupture and clot formation, stroke, heart attack and the like may be caused (Non-Patent Document 1).

There is scientific evidence that metabolic and behavioral risk factors heavily influence the aetiology of atherosclerosis. Behavioral risk factors include a lack of physical activity, unhealthy diet such as excessive calories, salt and fat intake, and tobacco and alcohol abuse. Traditional metabolic risk factors include hypertension, heightened blood lipids such as cholesterol, hyperglycemia. Additionally, increased carotid intima-media thickness (CIMT) has been reported to be associated with CVD risk factors.

The most well-known forms of diabetes are type 1 and type 2. Type 2 diabetes is generally regarded as a lifestyle-related disease associated with a chronic caloric surplus, lack of exercise, obesity and high serum levels of low-density lipoprotein (LDL). Type 2 diabetes has insulin resistance and/or insulin deficiency, and hyperglycemia (Non-Patent Document 2). It has been made clear that dysfunction of adipocytes associated with an over-accumulation of visceral fat in obesity, namely, abnormality of adipocytokine secretion induces insulin resistance, which plays an important role as various causes of pathological conditions of type 2 diabetes and arteriosclerotic diseases.

Macrophages being myeloid cells are always in visceral adipose tissue. On the other hand, adipocytokine (MCP-1) is secreted from visceral adipocytes having insulin resistance with enlargement, macrophages are mobilized from surrounding blood vessels. Further, a large number of free fatty acids secreted from enlarged adipose tissue activate macrophages through Toll-like receptors, and secrete inflammatory cytokines such as TNFα and IL-6. These inflammatory cytokines act on adipocytes, resulting in the negative effect of adipocyte insulin resistance. Thus, the abnormality of secreting adipocytokine is enhanced. In recent years, it has been found that chronic inflammation of these adipocytes evokes systemic insulin resistance, and the pathophysiology of adipose tissue has been noted (Non-Patent Document 3).

In recent years, type 2 diabetes has been typically diagnosed by means of hematology. In hematology, fasting blood glucose, HbA1c, glycoalbumin and the like are measured as an index. However, to fully grasp the condition of type 2 diabetes, a plurality of indices must be measured and evaluated, not only one index.

The inventors found that diseases having insulin-resistant conditions selected from the group consisting of type 2 diabetes, hyperlipidemia, hypertension and obesity can be detected by quantifying a ganglioside GM3 (α-Neu5Ac-(2-3)β-Gal-(1-4)-β-Glc-(1-1)-ceramide) which is one kind of ganglioside in the blood (Patent Document 1, Non-Patent Document 4). Gangliosides collectively refer to sphingoglycolipids containing sialic acid, and the sphingoglycolipids are glycolipids which are biosynthesized by enzymatic stepwise addition of sugars, or sulfuric acid, such as glucose, galactose, N-acetylgalactosamine, N-acetylglucosamine and sialic acid, to ceramide and are expressed outside the lipid bilayer of the cell membrane.

Further, it has been found that the total amount of GM3 is correlated with lipoprotein cholesterol (LDL-c) (Non-Patent Document 4).

There are a large number of species of ganglioside GM3, the diversity of which depends on the number of carbons or double bonds, as well as the type and number of substituents that comprise the ceramide moiety. Depending on the species, the biophysical properties and physiological functions of GM3 are different so that it has been thought that measuring a ganglioside GM3 with a specific structure is useful for detecting lifestyle-related diseases such as metabolic syndrome. In fact, to date, ganglioside GM3 with a specific structure useful for the detection of lifestyle-related diseases has not been reported.
JP H01-93562 discloses several derivatives of GM3 having a fatty acid residue carrying an OH group at the second position.
Riboni et al., 1992, Eur J Biochem, 203, 107-113 discloses several GM3 derivatives having a hydroxylated fatty acid residue and the fact that their concentration is associated with aging.
Ladisch et al., 1995, Biochem, 34(4), 1197-1202 discloses GM3 compounds having a structure falling within the scope of formula (1) of the present invention. In particular, the fatty acid residues carry an OH group at the second position. Mikami et al, 1992, Biochiom Biophys Acta, 1125(1), 104-109 discloses several derivatives of GM3, including compounds falling within the structure formula (1) of the present invention.

### PRIOR ART REFERENCES

### Patent Documents

Patent Document 1: WO 2007/139224

### Non Patent Documents

Non-Patent Document 1: Kwon, G. P. et al., Circulation, 117 (2008) 2919-2927
Non-Patent Document 2: Chen, L. et al., Nat rev Endocrinol., 8 (2012) 228-236
Non-Patent Document 3: Katada, H. et al., Chem Bio Chem., 10 (2009) 1279
Non-Patent Document 4: Sato, T. et al., Obe Res Clin Pract., 2 (2008) I-II

### SUMMARY OF THE INVENTION

### PROBLEMS TO BE SOLVED BY THE INVENTION

The present invention has been accomplished in view of the above-mentioned background art, and an object thereof is to provide a method for detecting diseases or detecting onset risk of these diseases, comprising a step of measuring a ganglioside GM3 with a specific structure which is correlated with a large number of molecules regarding lifestyle-related diseases (specific diseases) such as metabolic syndrome.

### MEANS TO SOLVE THE PROBLEMS

The present inventors have earnestly investigated to solve the above-mentioned problems, and as a result, they have found that one disease selected from the group consisting of visceral fat accumulation, hyperglycemia, dyslipidemia, hypertension and atherosclerosis or **a combination of two or more of said diseases or onset risk of these diseases can be** detected by measuring a ganglioside GM3 in which the ceramide moiety is hydroxylated.

They have further found that a ganglioside GM3 with the specific structure is correlated with a **marker** regarding at least one disease selected from the group consisting of visceral fat accumulation, hyperglycemia, dyslipidemia, hypertension and atherosclerosis or **a combination of two or more of said diseases, whereby they have accomplished the present invention.**

That is, the present invention is directed to
[1] A method for detecting at least one disease selected from the group consisting of visceral fat accumulation, hyperglycemia, dyslipidemia, hypertension and atherosclerosis or **a combination of two or more of said diseases or detecting onset risk of these diseases, which comprises**
   (a) a step of measuring a ganglioside GM3 in a blood sample derived from a subject,
      wherein the ganglioside GM3 has a structure represented by the following formula (1). 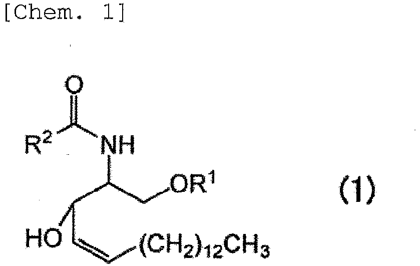
   [In the formula (1), R¹ represents a glycan constituting the ganglioside GM3, and R²-C(=O)- represents a fatty acid residue having 16 or more and 24 or less carbon atoms which may have a double bond, and having an OH group as a substituent.]
[2] The detection method according to [1], wherein the disease is metabolic syndrome.
[3] The detection method according to [1] or [2], wherein the structure represented by the formula (1) is a hydrocarbon group having 22 or 24 carbon atoms which may have a double bond, and R²-C(=O)- has an OH group as a substituent.
[4] The detection method according to [1] or [2], wherein the structure represented by the formula (1) is a hydrocarbon group having 24 carbon atoms which has a double bond, and R²-C(=O)-has an OH group as a substituent.
[5] The detection method according to [1] or [2], wherein the structure represented by the formula (1) is a structure represented by the following formula (2). 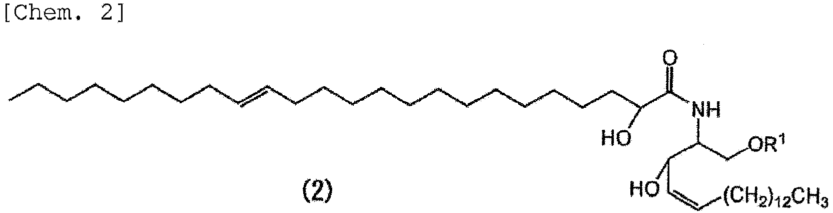 [In the formula (2), R¹ represents a glycan constituting the ganglioside GM3.]
[6] The detection method according to [5], wherein the method which further comprises,
   (b) a step of comparing an amount of the ganglioside GM3 with the structure represented by the formula (2) measured in the Step (a), with an amount of the ganglioside GM3 with the structure represented by the formula (2) in the blood sample derived from a normal subject,
   (c) a step of determining the value of the marker with regard to at least one disease selected from the group consisting of visceral fat accumulation, hyperglycemia, dyslipidemia, hypertension and atherosclerosis or **a combination of two or more of said diseases** of the subject, to be abnormal as compared with those of the normal subject, when there is a significant difference in the step (b),
   **wherein said marker is correlated with said ganglioside GM3.**
[7] The detection method according to [6], wherein the marker with regard to the visceral fat accumulation, hyperglycemia, dyslipidemia and hypertension is at least one marker selected from the group consisting of autotaxin, HOMA-IR, HbA1c, aspartic acid aminotransferase, alanine-aminotransferase, low-density lipoprotein cholesterol, fasting blood sugar and blood pressure.
[8] The detection method according to [6] or [7], wherein the marker with regard to the atherosclerosis is at least one selected from the group consisting of sphingomyelin, lysophosphatidylcholine, C-reactive protein and mean intima-media thickness.
[9] The detection method according to any of [1] to [8], wherein the measurement of the ganglioside GM3 is carried out by a method of reacting an antibody to the GM3, a high performance liquid chromatography method, mass spectrometry or liquid chromatography/mass spectrometry.

**Also disclosed herein is a detection kit, for detecting at least one disease** selected from the group consisting of visceral fat accumulation, hyperglycemia, dyslipidemia, hypertension and atherosclerosis or **a combination of two or more of said diseases**, or onset risk of these diseases, which contains a ganglioside GM3 as a standard substance,
wherein the ganglioside GM3 has a structure represented by the following formula (1). 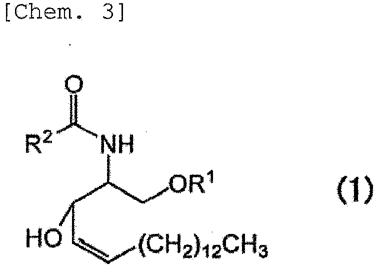 [In the formula (1), R¹ represents a glycan constituting the ganglioside GM3, and R²-C(=O)- represents a fatty acid residue having 16 or more and 24 or less carbon atoms which may have a double bond, and having an OH group as a substituent.]

### Effects of the invention

According to the detection method of the present invention, the above-mentioned problems can be solved, the above-mentioned tasks can be settled, and by measuring the ganglioside GM3 with a specific structure in a blood sample, the conditions of the subject can be grasped more accurately and more simply and easily from a more fundamental point of view, as compared with the conventional detection methods.

By measuring the specific ganglioside GM3 represented by the formula (1), a specific disease or the onset risk of the specific diseases can be detected more accurately as compared with the measurement of the general ganglioside GM3 (measurement of the entire ganglioside GM3).

Further, the conditions relating to a lifestyle-related disease of the subject can be detected more accurately as compared with the conventional detection method of the lifestyle-related diseases.

More specifically, according to the detection method of the present invention, "at least one disease selected from the group consisting of visceral fat accumulation, hyperglycemia, dyslipidemia, hypertension and atherosclerosis or a **combination of two or more of said diseases**" can be detected by measuring the ganglioside GM3 with a specific structure more accurately and more simply and easily from a more fundamental point of view, as compared with the conventional methods.

In the present specification, the above-mentioned matter in the double quotation marks (including the combined diseases) is sometimes abbreviated as "specific disease(s)".

It is also possible to predict onset risk of the specific diseases more accurately, more simply and easily at an earlier stage from a more fundamental point of view, as compared with the conventional methods, and is possible to take measures early so as not to suffer from the specific diseases, etc.

Further, according to the detection method of the present invention, even when the specific diseases cannot be detected by measuring the total GM3 amount, it is possible to detect the specific diseases, or to detect onset risk of the specific diseases.

In particular, according to the detection method of the present invention, it is possible to detect whether the subject is suffered from the metabolic syndrome or not, or to determine the onset risk.

In addition, the ganglioside GM3 with a specific structure has correlation with markers of visceral fat accumulation, hyperglycemia, dyslipidemia, hypertension and atherosclerosis, and it is possible to detect whether it is suffered from the specific diseases or to predict whether there is a risk of developing the specific disease, by the simple and easy operation that the ganglioside GM3 with a specific structure is measured.

According to the present invention, by detecting the specific diseases or the diseases such as metabolic syndrome, lifestyle-related disease, etc., or to detect onset risk of these diseases, detection and prevention of circulatory diseases, caused by them, such as diabetes; cardiovascular disorders such as cardiac infarction and angina; cerebrovascular disorders such as cerebral infarction; renal dysfunction; vascular disorders such as extremital artery obstruction; become easy.

### BRIEF DESCRIPTION OF DRAWINGS

[FIG. 1]

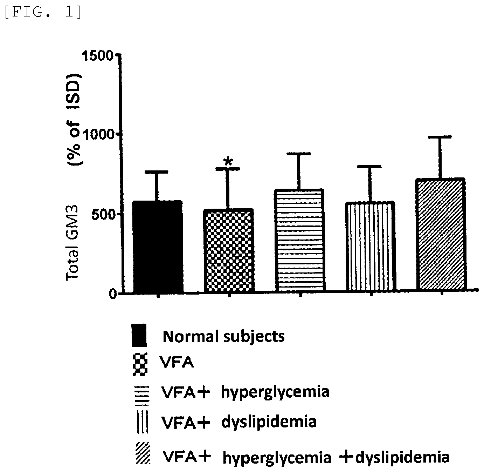

   FIG. 1 is a graph showing the measurement results of the total amount of GM3 in the serum of normal subjects and patients with each disease.
[FIG. 2]

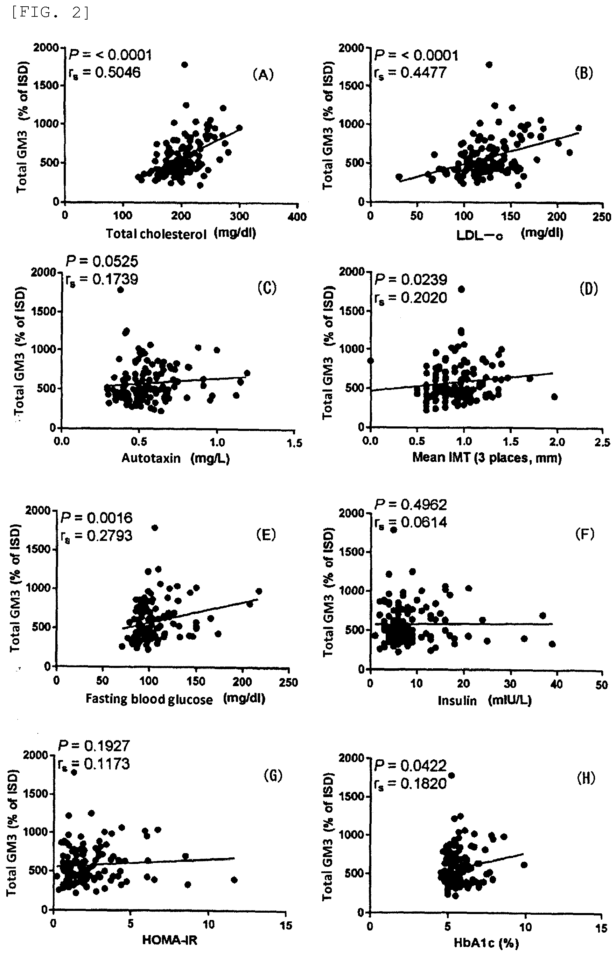

   FIG. 2 shows graphs showing the correlation between the total amount of GM3 in the serum, and (A) the total amount of cholesterol, (B) the amount of LDL-cholesterol (LDL-c), (C) the amount of autotaxin, (D) mean intima-media thickness (mean IMT), (E) fasting blood glucose, (F) insulin, (G) the amount of HOMA-IR or (H) the amount of HbA1c.
[FIG. 3A]

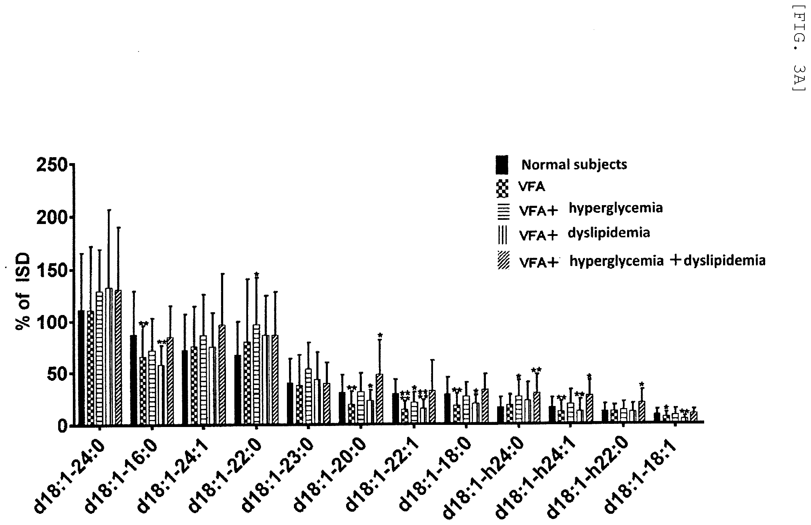

   FIG. 3A is a graph showing the measurement results of the amount of 12 kinds of GM3 molecular species, in the serum of normal subjects and patients with each disease.
[FIG. 3B]

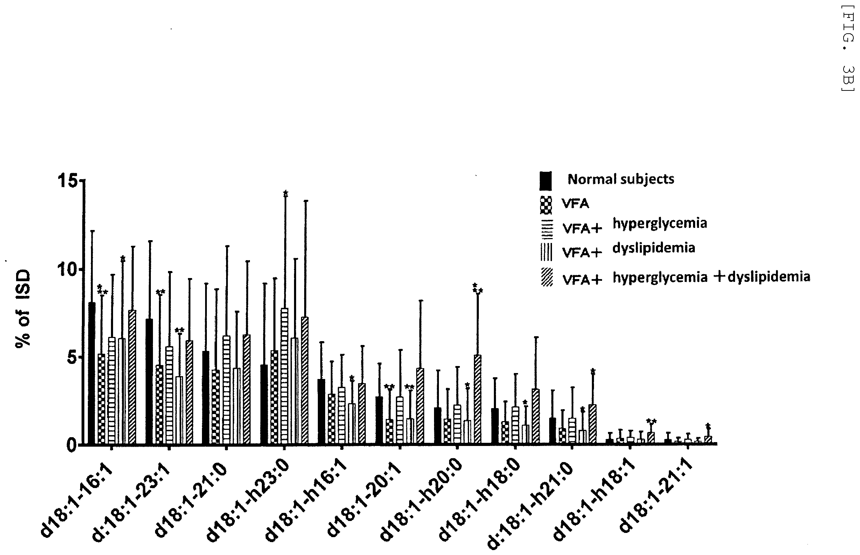

   FIG. 3B is a graph showing the measurement results of the amount of 11 kinds of GM3 molecular species, in the serum of normal subjects and patients with each disease.
[FIG. 4]

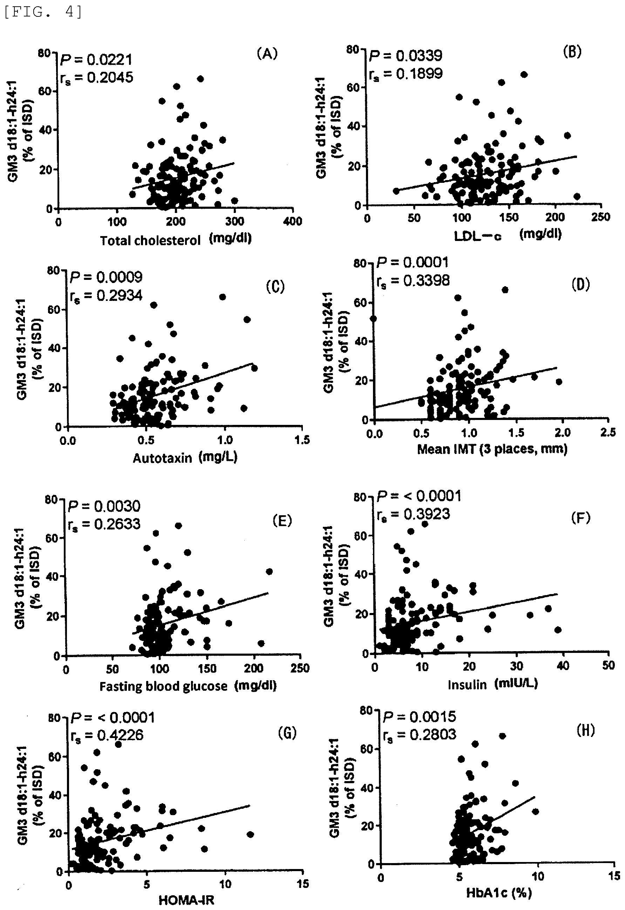

   FIG. 4 shows graphs showing the correlation between the amount of GM3 with a specific structure ([hC24:1]GM3) in the serum, and (A) the total amount of cholesterol, (B) the amount of LDL-cholesterol (LDL-c), (C) the amount of autotaxin, (D) mean intima-media thickness (mean IMT), (E) fasting blood glucose, (F) insulin, (G) the amount of HOMA-IR or (H) the amount of HbA1c.
[FIG. 5]

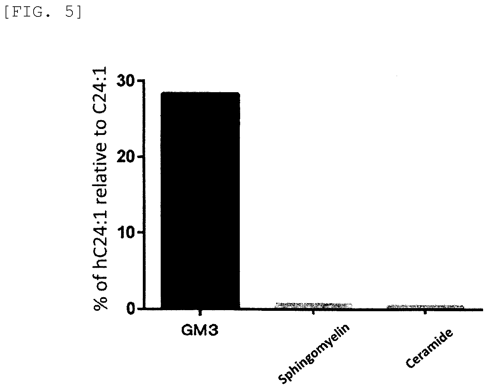

   FIG. 5 is a graph showing the measurements of the amount of [hC24:1] present in GM3, sphingomyelin or ceramide. The vertical axis shows the ratio (%) based on the amount of [C24:1] of 100.
[FIG. 6]

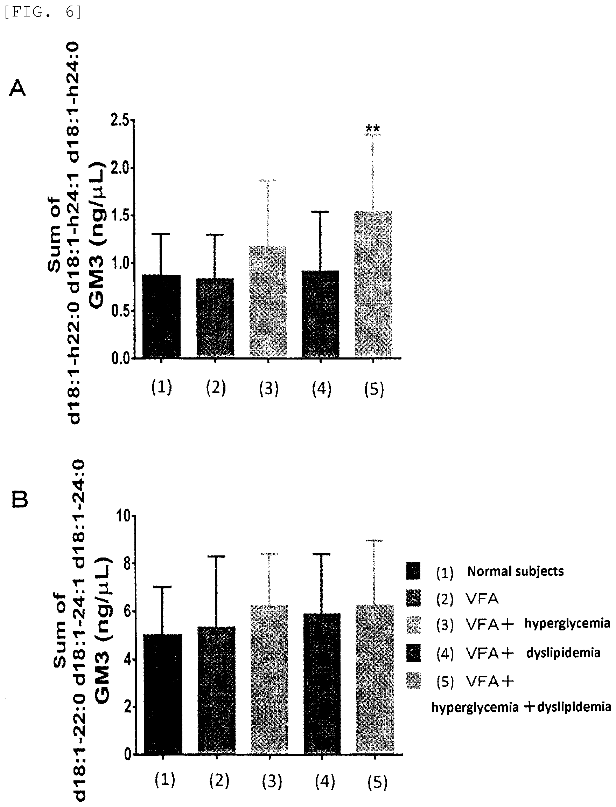

   FIG. 6 shows graphs showing the measurement results of (A) the total amount of hydroxylated GM3 ("d18:1-h24:0", "d18:1-h24:1", "d18:1-h22:0") in the serum, (B) the total amount of non-hydroxylated GM3 ("d18:1-24:0", "d18:1-24:1", "d18:1-22:0") in the serum, of normal subjects and patients with each disease.
[FIG. 7]

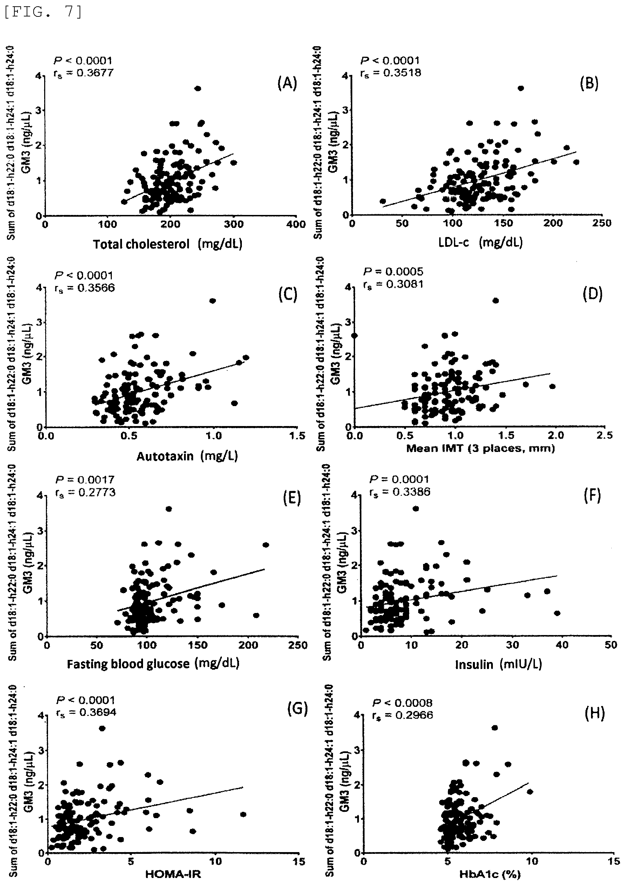

   FIG. 7 shows graphs showing the correlation between the total amount of hydroxylated GM3 ("d18:1-h24:0", "d18:1-h24:1", "d18:1-h22:0") in the serum, and (A) the total amount of cholesterol, (B) the amount of LDL-cholesterol (LDL-c), (C) the amount of autotaxin, (D) mean intima-media thickness (mean IMT), (E) fasting blood glucose, (F) insulin, (G) the amount of HOMA-IR or (H) the amount of HbA1c.
[FIG. 8]

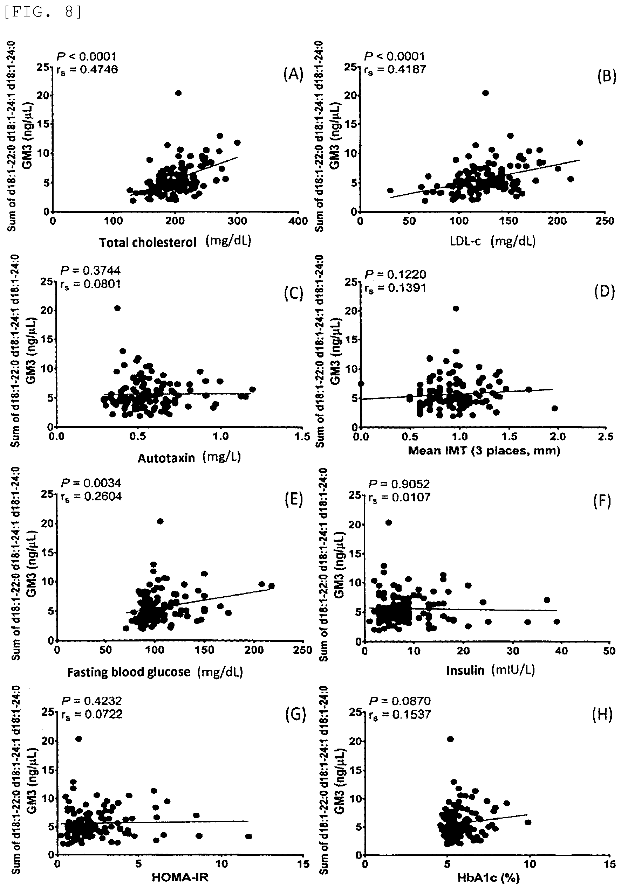

   FIG. 8 shows graphs showing the correlation between the total amount of non-hydroxylated GM3 ("d18:1-24:0", "d18:1-24:1", "d18:1-22:0") in the serum, and (A) the total amount of cholesterol, (B) the amount of LDL-cholesterol (LDL-c), (C) the amount of autotaxin, (D) mean intima-media thickness (mean IMT), (E) fasting blood glucose, (F) insulin, (G) the amount of HOMA-IR or (H) the amount of HbA1c.

### Embodiments to carry out the invention

Hereinafter, the present invention will be described.

The detection method of the present invention is a method for detecting at least one disease selected from the group consisting of visceral fat accumulation, hyperglycemia, dyslipidemia, hypertension and atherosclerosis or a disease two or more of which are combined, or detecting onset risk of these diseases which comprises
(a) a step of measuring a ganglioside GM3 in a blood sample derived from a subject, wherein the ganglioside GM3 has a structure represented by the following formula (1). 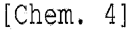 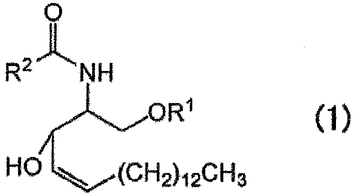 [In the formula (1), R¹ represents a glycan constituting the ganglioside GM3, and R²-C(=O)- represents a fatty acid residue having 16 or more and 24 or less carbon atoms which may have a double bond, and having an OH group as a substituent.]

In the present specification, each disease is defined as follows.
<1> When a waist size at the position of the navel of a subject is 85 cm or more in the case of male, and 90 cm or more in the case of female, it is determined to be visceral fat accumulation (hereinafter sometimes abbreviated as "VFA").
<2> When a fasting blood sugar level of the subject is 126 mg/dL or more, it is determined to be hyperglycemia.
<3> When one satisfies at least one condition among the following conditions (1) to (3), it is determined to be dyslipidemia.
   (1) A triglyceride (neutral fat) value of the subject is 150 mg/dL or more.
   (2) A low-density lipoprotein cholesterol (LDL-c) value of the subject is 140 mg/dL or more.
   (3) A high-density lipoprotein cholesterol (HDL-c) value of the subject is less than 40 mg/dL.
<4> When a systolic blood pressure (the maximum blood pressure) of the subject is **18.7 kPa (140 mmHg) or higher and/or a diastolic blood pressure (the minimal blood pressure) thereof is 12 kPa (90 mmHg) or** higher, it is determined to be hypertension.
<5> Atherosclerosis is also referred to as atheromatous arteriosclerosis, and refers to the state in which plaque (porridge species) is formed at an inner membrane of a blood vessel by accumulating fat or cholesterol at the inner membrane of the blood vessel.
<6> Nephropathy is a disease in which kidney function is lowered. There is nephropathy which is generated by causing disorder at the capillaries of the kidney by continuing the state of a high blood glucose level due to hyperglycemia, etc.
<7> In addition, the diseases considered to be caused by lifestyle habit such as the VFA, hyperglycemia, dyslipidemia, hypertension, atherosclerosis, etc., are sometimes abbreviated as "lifestyle-related disease".

The detection method of the present invention is a method for detecting at least one disease selected from the group consisting of visceral fat accumulation, hyperglycemia, dyslipidemia, hypertension and atherosclerosis or **a combination of two or more of said diseases**, or for detecting onset risk of these diseases.

"To detect the disease two or more of which are combined" means "to detect whether or not the subject is developed at least two or more diseases selected from the group consisting of visceral fat accumulation, hyperglycemia, dyslipidemia, hypertension and atherosclerosis".

Further, "to detect onset risk of the disease **or a combination of two or more of said diseases" means "to detect whether or not the** subject has onset risk of at least two or more diseases selected from the group consisting of visceral fat accumulation, hyperglycemia, dyslipidemia, hypertension and atherosclerosis".

The detection method of the present invention is a detection method of a specific disease or a detection method of onset risk of the specific disease, and the detection method of the present invention is also a method for detecting a lifestyle-related disease, or for detecting onset risk of the lifestyle-related disease.

Moreover, the detection method of the present invention is also a method for detecting metabolic syndrome, or for detecting onset risk of the metabolic syndrome.

In the present specification, the "metabolic syndrome" refers to the state in which the subject is in visceral fat accumulation, and is developed at least two diseases among "hyperglycemia, dyslipidemia and hypertension". Therefore, the state of the "metabolic syndrome" is included in the state that the subject is developed specific diseases.

### <Step (a)>

In the step (a), the specific ganglioside GM3 with a structure represented by the following formula (1) in the blood sample derived from the subject is measured. The "measurement" is a concept that includes to confirm the presence of the specific ganglioside GM3 in the blood, or to quantitate the specific ganglioside GM3 in the blood. The "measurement" may further comprise other steps, if necessary.

In general, the term "Ganglioside" refers to a glycosphingolipid in which one or more sialic acid is bound onto the glycan.

The terms "ganglioside GM3" means that the glycan of the glycosphingolipid is "α-Neu5Ac-(2-3)β-Gal-(1-4)-β-Glc-(1-1)-ceramide".

Here, "α-Neu5Ac" means N-acetyl-α-neuraminic acid, "β-Gal" means β-galactose, and "β-Glc" means β-glucose.

The ganglioside GM3 (hereinafter, it is sometimes simply abbreviated as "GM3") to be measured in the present invention is a GM3 with the structure particularly represented by the following formula (1). 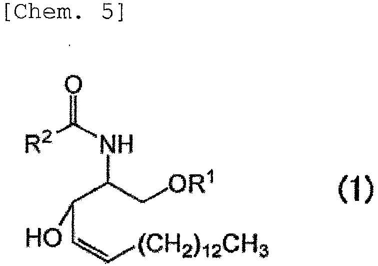 [In the formula (1), R¹ represents a glycan constituting the ganglioside GM3, and R²-C(=O)- represents a fatty acid residue having 16 or more and 24 or less carbon atoms which may have a double bond, and having an OH group as a substituent.]

Here, from the definition of the "ganglioside GM3", "R¹ which is a glycan constituting the ganglioside GM3" in the formula (1) means that "α-Neu5Ac", "β-Gal" and "β-Glc" are bonded as in the definition of the "ganglioside GM3".

Hereinafter, the "GM3 with the structure represented by the formula (1)" is sometimes abbreviated simply as "hydroxylated GM3".

With regard to the structure represented by the formula (1), "R²-C(=O)-" in the formula (1) is preferably a hydrocarbon group having 22 or 24 carbon atoms which may have a double bond and having an OH group as a substituent in the points that detection of the specific disease can be carried out more accurately, and detection of onset risk of the specific disease can be carried out more accurately as compared with the measurement of the entire GM3 such as quantification of the total amount of GM3.

Also, it is more preferable that R² is a hydrocarbon group having 23 carbon atoms which has a double bond, and having an OH group as a substituent from the above-mentioned points.

Further, it is particularly preferable that the structure represented by the formula (1) is a structure represented by the following formula (2) from the above-mentioned points. 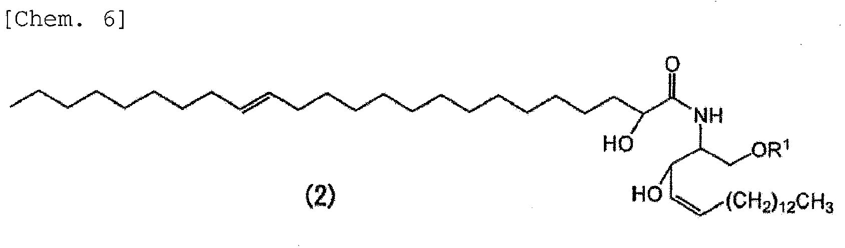 [In the formula (2), R¹ represents a glycan constituting the ganglioside GM3.]

Hereinafter, "GM3 with a structure represented by the formula. (2)" is sometimes abbreviated as "[hC24:1]GM3". The " [hC24 :1] GM3" is a fatty acid having 24 carbon atoms (the carbon to which the oxygen of the carboxyl group of the fatty acid is bonded by a double bond is counted as one.) in which the fatty acid of GM3 has an OH group as a substituent, and it means that it has a double bond in the hydrocarbon group.

The "subject" in the present invention represents mammals such as humans, dogs, cats, horses, cows, pigs, sheep and monkeys, and the subject is preferably humans.

For example, "blood sample derived from the subject" to be used in the present invention is whole blood, serum, and plasma. It is preferable to use "serum or plasma derived from the subject" in the point that highly accurate measurement can be carried out.

For example, the whole blood collected from the subject is charged in a blood-collecting tube to which an anticoagulant is added, and after mixing with inversion, it is centrifuged whereby plasma can be recovered. Also, after coagulating the whole blood collected from the subject, it is centrifuged whereby serum can be recovered.

The extraction method of the "GM3 with the specific structure represented by the formula (1)" in the blood sample can use conventionally known methods. More specifically, the GM3 can be extracted from the blood sample by the method described in Examples described below, etc.

In addition, the detection method of the present invention is preferably a detection method which further comprises,
(b) a step of comparing an amount of the ganglioside GM3 with the structure represented by the formula (2) measured in the step (a), with an amount of the ganglioside GM3 with the structure represented by the formula (2) in the blood sample derived from a normal subject,
(c) a step of determining the value of the marker with regard to at least one disease selected from the group consisting of visceral fat accumulation, hyperglycemia, dyslipidemia, hypertension and atherosclerosis or **a combination of two or more of said diseases** of the subject, to be abnormal as compared with those of the normal subject, when there is a significant difference in the step (b).

### <Step (b)>

In the step (b), the amount of the ganglioside GM3 with the structure represented by the formula (2) measured in the step (a), and the amount of the ganglioside GM3 with the structure represented by the formula (2) in the blood sample derived from a normal subject are compared with each other.

### <Step (c)>

In the step (c), when there is a significant difference in the step (b), the value of the marker with regard to at least one disease selected from the group consisting of visceral fat accumulation, hyperglycemia, dyslipidemia, hypertension and atherosclerosis or **a combination of two or more of said diseases** of the subject is determined to be abnormal as compared with those the normal subject.

The "significant difference" means when the amount of the ganglioside GM3 with the structure represented by the formula (2) in the blood sample derived from the subject, and the amount of the ganglioside GM3 with the structure represented by the formula (2) in the blood sample derived from the normal subject are subjected to statistical analysis by using Mann-Whitney U test, two-sample t-test or the like, and as a result, the P value is 0.05 or less.

When the lower limit of the value (fold) dividing the value of the ganglioside GM3 with the structure represented by the formula (2) in the blood sample derived from the subject, by the value of the ganglioside GM3 with the structure represented by the formula (2) in the blood sample derived from the normal subject is within the range of 1.2-fold or more to 2.5-fold or more, it is preferable to determine the marker value of the specific disease to be abnormal, when it is within the range of 1.4-fold or more to 2.3-fold or more, it is more preferable to determine the same to be abnormal, and when it is within the range of 1.6-fold or more to 2-fold or more, it is particularly preferable to determine the same to be abnormal. If the lower limit of lower limit value of the divided value is too low, there is sometimes a case where the normal subject is determined to be abnormal, while if the upper limit of the lower limit of the divided value is too high, there is sometimes a case where it does not determine to be abnormal even when it is the abnormal case.

The "marker with regard to the disease in which two or more are combined" means the "marker with regard to at least two or more diseases selected from the group consisting of visceral fat accumulation, hyperglycemia, dyslipidemia, hypertension and atherosclerosis".

The step (c) is based on the fact that when the GM3 with the structure represented by the formula (1) is in particular the ganglioside GM3 with the structure represented by the formula (2) ([hC24:1]GM3), the inventors have found that it had a correlation with the marker (biomarker) regarding the specific disease.

That is, for example, a particularly preferable method is that, by measuring one molecule as [hC24:1]GM3, "the value of the marker of the specific disease" which has a correlation with the [hC24:1]GM3 can be predicted, whether the value of the marker is abnormal or not as compared with the normal subject can be estimated or determined, and based on such a determination, the specific disease is detected, or onset risk of the specific disease is detected.

The marker with regard to the visceral fat accumulation, hyperglycemia, dyslipidemia and hypertension is preferably at least one marker selected from the group consisting of autotaxin, HOMA-IR (Homeostasis model assessment-Insulin Resistance), HbA1c (Hemoglobin A1c), aspartic acid aminotransferase (AST), alanine-aminotransferase (ALT), low-density lipoprotein cholesterol (LDL-c), fasting blood sugar and blood pressure.

The marker with regard to the atherosclerosis is preferably at least one marker selected from the group consisting of sphingomyelin, lysophosphatidylcholine, C-reactive protein and mean intima-media thickness (IMT).

The measurement method of the specific ganglioside GM3 is not particularly limited, and it is preferable to use a method of reacting an antibody to the GM3, a high performance liquid chromatography method, mass spectrometry or liquid chromatography/mass spectrometry. These methods may be used in combination.

The method of acting an antibody to the GM3 is not particularly limited, and is ELISA method, for example.

The high performance liquid chromatography method (hereinafter sometimes abbreviated as "HPLC") is not particularly limited and may be used, for example, normal phase chromatography, reverse phase chromatography, ion exchange chromatography or the like.

The mass spectrometry is not particularly limited and may be used, for example, as an ionization method, MALDI method, ESI method, APCI method or the like. Also, as a separating portion, a time-of-flight type (TOF type), a double focusing type, a quadrupole focusing type, an ion trap type or the like may be used. In the measurement (detection, quantification) of the GM3 with the specific structure, it is preferable to use the ESI method or the APCI method as the ionization method, and it is preferable to use the quadrupole focusing type as the separating portion.

The "liquid chromatography/mass spectrometry (LC/MS) " is an analytical method in which the high performance liquid chromatography method and the mass spectrometry are combined.

### <Detection kit>

The present **disclosure** further provides a detection kit, for detecting at least one disease selected from the group consisting of visceral fat accumulation, hyperglycemia, dyslipidemia, hypertension and atherosclerosis or **a combination of two or more of said diseases**, or onset risk of these diseases, which contains a ganglioside GM3 as a standard substance, wherein the ganglioside GM3 has a specific structure represented by the following formula (1). 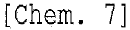 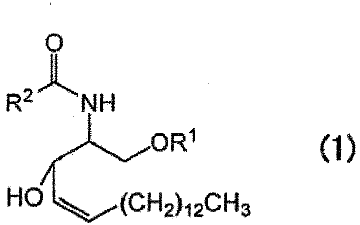 [In the formula (1), R¹ represents a glycan constituting the ganglioside GM3, and R²-C(=O)- represents a fatty acid residue having 16 or more and 24 or less carbon atoms which may have a double bond, and having an OH group as a substituent.]

When the detection kit of the present **disclosure** is used, the above-mentioned effects can be more suitably exerted. That is, when the detection kit of the present invention is used, by using the detection method of the present **disclosure**, it is easily possible to detect at least one disease selected from the group consisting of hyperglycemia, dyslipidemia, hypertension and atherosclerosis or **a combination of two or more of said diseases**, or to detect onset risk of these diseases.

Further, when the detection kit of the present **disclosure** is used, it is possible to detect whether or not the subject is suffering from a specific disease such as lifestyle-related diseases, etc., or to detect whether or not the subject has a risk of developing a specific disease such as lifestyle-related disease, etc., whereby the state of the subject can be more accurately grasped.

### EXAMPLES

Hereinafter, the present invention will be explained more specifically by showing Examples.

### <Subjects>

Subjects were 125 Japanese people. Among the 125 people, normal subjects were 26, patients with only visceral fat accumulation (VFA) were 39, patients with VFA and hyperglycemia were 15, and patients with VFA and dyslipidemia were 28. Patients with VFA, hyperglycemia and dyslipidemia, namely patients with metabolic syndrome were 17.

All subjects gave their written informed consent prior to their inclusion in the study.

### <Extraction of lipids>

100 ng of ¹³C-labeled GM3 (hereinafter abbreviated as "d18:1-[¹³C]16:0") was added for an internal standard to 50 µL of serum, the solution was lyophilized, and then dissolved in 5 mL of a mixture of chloroform and methanol (chloroform : methanol=1:1). The mixture was sonicated, incubated at 40°C for 1 hour, and then centrifuged at 4°C for 30 minutes (15,000×g). The supernatant was collected and the pellet was subjected to the same extraction procedure. Obtained supernatants were combined and evaporated, thereby extracting lipids from the serum.

### <Mass Spectrometry>

Each GM3 species was measured using LC-MS/MS with electrospray ionization, in multiple reaction monitoring negative ionization mode. Accela 1250 Pump (Thermo Fisher Scientific Inc.) as LC and Vantage AM (quadrupole type, Thermo Fisher Scientific Inc.) as a mass spectrometry device were used. Calibration of the mass spectrometry device was performed using a mixture of GM3 species extracted from milk.

Lipids extracted from 50 µL of serum were dissolved in 50 µL of methanol, and 6 µL of the mixture was injected into LC-MS/MS. In the 6 µL of the mixture, 12 ng of d18:1-[¹³C]16:0 being the internal standard was contained.

The settings of LC are as follows.
(1) Column: Develosil C30-UG-1 (50mm, Nomura Chemical Co., Ltd.)
(2) Flow rate: 50 µL/min
(3) Mobile phase: (i) A mixture of 20% of H₂O, 50% of 2-propanol and 30% of methanol, containing 0.1% acetic acid and 0.1% ammonia (hereinafter referred to as "solvent A"). (ii) A mixture of 2% of H₂O, 50% of 2-propanol and 48% of methanol, containing 0.1% acetic acid and 0.1% ammonia (hereinafter referred to as "solvent B").
(4) Gradient program: The gradient program employed started with 100% solvent A for 5 min then ramped to 100% solvent B over 30 min. 100% solvent B was maintained for 4 min, then the solvent was returned to 100% solvent A over 1 min and held there for 10 min.

The settings of mass spectrometry device are as follows.
(1) Spray voltage: -2500 V
(2) Capillary temperature: 204°C
(3) Vaporizer temperature: 373°C
(4) Sheath gas pressure (nitrogen gas): 50 (arbitrary units)
(5) Ion sweep gas pressure: 0 (arbitrary units)
(6) Auxiliary gas pressure (nitrogen gas) : 15 (arbitrary units)
(7) S-lens RF amplitude: 276
(8) Ionic dissociation (CID) collision energy: 0
(9) Collision pressure: 1.0 mTorr
(10) Collision energy: 53 eV
(11) Scan time: 0.01 second

The amount of each GM3 species was quantified based on the internal standard (d18:1-[¹³C]16:0). The total amount of GM3 was calculated based on the total amount of 23 kinds of GM3 detected.

### <Statistical analysis>

The results of quantitative analysis were shown as mean±SD. For all statistical analysis, P≤0.05 was deemed statistically significant. In statistical analysis, Mann-Whitney U test or two-sample t-test was used to obtain Spearman's rank correlation coefficients.

Further, in Example 5, all GM3 species were subjected to multivariate analysis. Body mass index (BMI), waist, calcaneus stiffness, diastolic blood pressure (DBP), systolic blood pressure (SBP), Brinkman index, ejection fraction, HOMA-IR, hemoglobin (HbA1c), platelets, AST (glutamic oxaloacetic transaminase (GOT)), ALT (glutamate-pyruvate transaminase (GPT)), γ-glutamyl transpeptidase (y-GTP), triglyceride, creatinine, uric acid, high-density lipoprotein cholesterol (HDL-c), C-reactive protein, LDL-c, free fatty acids, total adiponectin, autotaxin, sphingomyelin, phosphotidylcholine, lysophosphatidylcholine, and IMT were investigated as explanatory variables. All statistical analysis were conducted using GraphPad Prism 6.04 (GraphPad Software Inc.) except multivariate analysis when XLSTAT (version 2014.4.02, Addinsoft Corporation) was utilized.

### Example 1

### <Correlation between markers and each disease>

Markers (indices) with regard to specific diseases such as metabolic syndrome in (1) normal subjects (n=26), (2) patients with only VFA (n=39), (3) patients with VFA and hyperglycemia (n=15), (4) patients with VFA and dyslipidemia (n=28), and (5) patients with VFA, hyperglycemia and dyslipidemia (namely, patients with metabolic syndrome) (n=17) were measured, respectively. The results are shown in Table 1. In Table 1, (1) shows normal subjects, (2) shows patients with only visceral fat accumulation, (3) shows patients with visceral fat accumulation and hyperglycemia, (4) shows patients with visceral fat accumulation and dyslipidemia, and (5) shows patients with visceral fat accumulation, hyperglycemia and dyslipidemia.

Further, in the table, "ns" shows P>0.05, "*" shows P≤0.05, "**" shows P≤0.01, "***" shows P≤0.001, "****" shows 0.0001. All pieces of data in the table are reported as mean±SD.

### [Table 1]

**Table 1**

| | (1) | (2) | (3) | (4) | (5) |
|---|---|---|---|---|---|
| Number of subjects | 26 | 39 | 15 | 28 | 17 |
| Sex (men/women) | 8/18 | 29/10 | 13/2 | 25/3 | 16/1 |
| Age | 51.8 ± 1.8 | 52.1 ± 1.5 ns | 59.9 ± 2.1 ** | 50.1 ± 1.6 ns | 54.4 ± 2.4 ns |
| BMI (kg/m²) | 24.3 ± 0.2 | 27.5 ± 0.4 **** | 27.3 ± 0.8 **** | 28.1 ± 0.6 **** | 28.8 ± 0.8 **** |
| Height (cm) | 159.6 ± 1.5 | 168.2 ± 1.5 *** | 167.4 ± 1.6 ** | 169.0 ± 1.5 **** | 168.8 ± 1.8 *** |
| Weight (kg) | 62.1 ± 1.1 | 78.0 ± 1.7 **** | 76.5 ± 2.7 **** | 80.3 ± 2.2 **** | 82.3 ± 2.8 **** |
| Waist (cm) | 83.3 ± 0.7 | 95.1 ± 1.0 **** | 96.0 ± 2.2 **** | 95.5 ± 1.5 **** | 97.3 ± 1.7 **** |
| SBP (mmHg)* | 110.1 ± 1.9 | 113.7 ± 2.0 ns | 121.0 ± 4.7 * | 114.4 ± 2.5 ns | 128.4 ± 3.1 **** |
| DBP (mmHg)* | 71.4 ± 1.1 | 72.2 ± 1.3 ns | 74.8 ± 2.9 ns | 75.4 ± 1.7 ns | 82.6 ± 2.1 **** |
| TG neutral lipids (mg/dL) | 85.1 ± 5.4 | 101.6 ± 3.8 * | 96.9 ± 7.0 ns | 266.5 ± 33.5 **** | 275.7 ± 35.0 **** |
| HDL-c (mg/dL) | 66.4 ± 2.5 | 54.2 ± 1.8 *** | 57.0 ± 2.9 * | 46.0 ± 1.8 **** | 43.0 ± 2.3 **** |
| LDL-c (mg/dL) | 121.4 ± 5.0 | 122.4 ± 4.5 ns | 130.3 ± 5.8 ns | 128.3 ± 7.5 ns | 132.8 ± 8.9 ns |
| Blood glucose (mg/dL) | 92.2 ± 1.4 | 94.6 ± 1.2 ns | 143 . 6 ± 8.4 **** | 96.3 ± 1.2 * | 127.2 ± 4.3 **** |
| Total cholesterol (mg/dL) | 201.6 ± 5.3 | 192.6 ± 4.7 ns | 204.5 ± 7.1 ns | 208.6 ± 7.6 ns | 210.7 ± 7.9 ns |
| Free fatty acid (µEq/L) | 540.6 ± 53.8 | 470.3 ± 38.1 ns | 444.5 + 49.5 ns | 527.7 ± 32.8 ns | 410.8 ± 43.2 ns |
| Insulin (µU/mL) | 5.0 ± 0.5 | 7.2 ± 0.7 * | 9.5 ± 2.0 ** | 10.7 ± 1.7 ** | 12.8 ± 1.2 **** |
| HOMA IR (mU/L) | 1.1 ± 0.1 | 1.7 ± 0.2 * | 3.4 ± 0.7 *** | 2.5 ± 0.4 ** | 4.0 ± 0.4 **** |
| HOMA *β* (%) | 64.5 ± 6.8 | 93.0 ± 16.0 ns | 46.4 ± 9.7 ns | 121.3 ± 21.6 * | 77.2 ± 8.2 ns |

| | | | | | |
|---|---|---|---|---|---|
| * **1 mmHg = 133 Pa** | | | | | |

### Example 2

### <Comparison of the total amount of GM3 in each disease>

The total amount of GM3 of (1) normal subjects (n=26), (2) patients with only VFA (n=39), (3) patients with VFA and hyperglycemia (n=15), (4) patients with VFA and dyslipidemia (n=28), and (5) patients with VFA, hyperglycemia and dyslipidemia (namely, patients with metabolic syndrome) (n=17) was measured, respectively. The results are shown in Fig. 1. The vertical axis of Fig. 1 shows the total amount (%) of GM3 based on the amount of internal standard ("d18:1-[¹³C]16:0") contained in the serum sample of 100%. Further, in the figure, "*" shows P<0.05.

The results of Fig. 1 showed that, the total amount of GM3 of (2) patients with only VFA was statistically significant compared with that of normal subjects, on the other hand, the total amount of GM3 of each of (3) patients with VFA and hyperglycemia, (4) patients with visceral fat accumulation and dyslipidemia, and (5) patients with visceral fat accumulation, hyperglycemia and dyslipidemia was not statistically significant compared with that of normal subjects.

### Example 3

### <Correlation between total GM3 and each disease marker>

Correlation between total GM3 and each disease marker (total cholesterol, LDL-c, autotaxin, mean IMT, fasting blood glucose, insulin, HOMA-IR, HbA1c) was analyzed using Spearman's rank correlation. The results are shown in Fig. 2.

As shown in Fig. 2, the total amount of GM3 was correlated with total cholesterol, LDL-c, mean IMT, fasting blood glucose, and HbA1c, respectively, on the other hand, not correlated with autotaxin, insulin, and HOMA-IR.

### <Results of Examples 2 and 3>

The results of Examples 2 and 3 suggested that, the possibility to be or become afflicted by specific diseases such as metabolic syndrome was not precisely determined by measuring or comparing the total amount of GM3.

### Example 4

### <Association between each GM3 species and lifestyle-related diseases>

Regarding GM3, there are a large number of molecular species depending on carbon numbers, the number of double bonds, presence or absence of substituents, or kinds of substituents, in a hydrocarbon group of the ceramide moiety. On the basis of results of Examples 2 and 3, it was thought that measuring specific GM3 molecular species can provide more precise information on subjects relating to lifestyle-related diseases or specific diseases, not measuring the total amount of GM3, so that correlation between each GM3 species and disease was analyzed.

Similar to Example 1, the amount of each GM3 species of (1) normal subjects (n=26), (2) patients with only VFA (n=39), (3) patients with VFA and hyperglycemia (n=15), (4) patients with VFA and dyslipidemia (n=28), and (5) patients with VFA, hyperglycemia and dyslipidemia (namely, patients with metabolic syndrome) (n=17) was measured. The results are shown in Figs. 3A and 3B.

In the horizontal axis of Fig. 3A, GM3 species are arranged in descending order of content in GM3 in the serum, from "d18:1-24:0" which is the most abundant species. In the horizontal axis of Fig. 3B, GM3 species are arranged in descending order of content in GM3 in the serum, from "d18:1-16:1" which is the 13th most abundant species.

Regarding the structure of each GM3 species, for example, in a case of "d18:1-24:0, " "d18:1" means that the sphingomyelin moiety of GM3 is a hydrocarbon group having 18 carbon atoms and the hydrocarbon group has a double bond. "24:0" means that the fatty acid of GM3 is a hydrocarbon group having 24 carbon atoms and the hydrocarbon group has no double bond. For example, when "d18:1-24 : 0" has an OH group as a substituent in the fatty acid, this is described as "d18:1-h24:0".

The vertical axis of Fig. 3 shows the amount (%) of each GM3 species, when the amount of internal standard ("d18:1-[¹³C]16:0") contained in the serum sample is 100%. Further, in the table, "*" shows P≤0.05, "**" shows P≤0.01, "***" shows P≤0.001, "****" shows P<0.0001. All pieces of data in the table are reported as mean±SD. Mann-Whitney U test was used.

The results of Fig. 3 showed that, many GM3 species ( "d18:1-h24:0", "d18:1-h24:1", "d18:1-h22:0", "d18:1-h20:0", "d18:1-h21:0", "d18:1-h18:1") having an OH group as a substituent in the fatty acid of GM3 of (5) patients with VFA, hyperglycemia and dyslipidemia was statistically significant compared with that of normal subjects. Further, "d18:1-20:0" and "d18:1-21:1" of (5) patients with VFA, hyperglycemia and dyslipidemia was statistically significant compared with that of normal subjects.

Particularly, "d18:1-h24:0", "d18:1-h24:1", "d18:1-h22:0" of (5) patients with VFA, hyperglycemia and dyslipidemia was statistically significant, with levels 1.7 times or more higher than that of normal subjects.

These results suggested that, GM3 having an OH group as a substituent in the fatty acid of GM3 (hydroxylated GM3) was useful for detecting specific diseases or lifestyle-related diseases such as metabolic syndrome.

### Example 5

### <Correlation between each GM3 species and disease marker>

Correlation between each disease marker, and GM3 species which comprised a large proportion of GM3 in the serum (12 kinds described in the horizontal axis of Fig. 3A) was analyzed using multivariate analysis. The results are shown in Table 2.

The disease markers are as follows.
(1) Marker for obesity: BMI
(2) Marker for metabolic syndrome: LDL-c, autotaxin (in Table 2, abbreviated as "ATX"), HOMA-IR, AST, ALT, HbA1c
(3) Marker for atherosclerosis: lysophosphatidylcholine (in Table 2, abbreviated as "LPC"), sphingomyelin (in Table 2, abbreviated as "SM"), ejection fraction (in Table 2, abbreviated as "EF"), C-reactive protein (in Table 2, abbreviated as "CRP"), platelets, triglyceride (TG)
(4) Markers for hypertension: blood pressure (BP)
(5) Markers for nephropathy: creatinine (CRE)
(6) Other markers: age

### [Table 2]

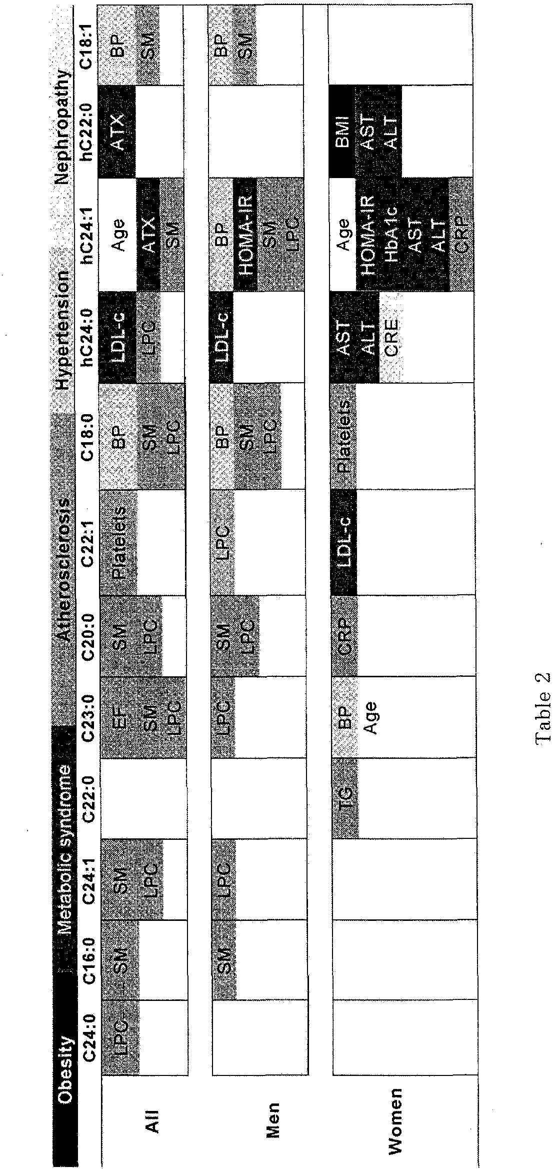

"C24:0" in Table 2 corresponds to "d18:1-24:0" in Fig. 3A. Similar to Fig. 3A, GM3 species were arranged in descending order of content of GM3 in the serum.

The results of Table 2 showed that hydroxylated GM3 (hC24: 0, hC24:1, hC22: 0) was correlated with makers with regard to specific disease more than non-hydroxylated GM3. Particularly, "hC24:1" with a structure represented by the following formula (2) ([hC24:1]GM3) was correlated with a large number of markers such as age, autotaxin, sphingomyelin, blood pressure, HOMA-IR, lysophosphatidylcholine, HbA1c, AST, ALT, C-reactive protein, and thus it was suggested that "hC24:1" was useful for detecting or diagnosing specific diseases.

Further, the results of Table 2 showed that all GM3 species were not correlated with molecules regarding specific disease. Since it is thought that the total amount of GM3 is increased with increase of molecular species not regarding specific disease, it is suggested that using the total amount of GM3 as a maker for detecting or diagnosing specific disease may induce false positive or erroneous detection or diagnosis. 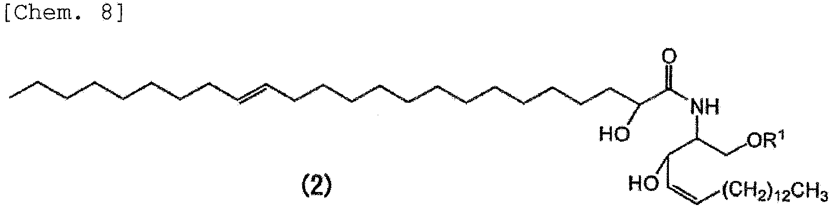 [In the formula (2), R¹ represents a glycan constituting the ganglioside GM3.]

### Example 6

### <Correlation between [hC24:1]GM3 and each disease marker>

The results of Example 5 suggested that [hC24:1]GM3 was useful as a marker for specific diseases such as metabolic syndrome, or a maker for lifestyle-related diseases or specific diseases. Next, correlation between [hC24:1]GM3 and each disease marker (total cholesterol, LDL-c, autotaxin, IMT, fasting blood glucose, insulin, HOMA-IR, HbA1c) was analyzed using Spearman's rank correlation. The results are shown in Fig. 4.

The results of Fig. 4 showed that the amount of [hC24:1]GM3 was correlated with total cholesterol, LDL-c, autotaxin, IMT, fasting blood glucose, insulin, HOMA-IR and HbA1c, respectively.

### Example 7

### <Measurement of content of [hC24:1]>

The amount of [hC24:1] relative to [C24:1] contained in each of the whole GM3, the whole sphingomyelin and the whole ceramide in the serum was measured. The results are shown in Fig. 5.

The vertical axis of Fig. 5 shows the ratio of the amount of "a fatty acid having 24 carbon atoms, having a hydroxyl group and having a double bond in the fatty acid" (abbreviated as [hC24:1]), to the amount of "a fatty acid having 24 carbon atoms and having a double bond in the fatty acid" (abbreviated as [C24:1]).

The results of Fig. 5 showed that 28.31% of [C24:1] contained in GM3 was [hC24:1].

On the other hand, 0.67% of [C24:1] contained in sphingomyelin was [hC24:1], and 0.45% of [C24:1] contained in ceramide was [hC24:1].

These results demonstrated that, on the basis of the fact that patients with specific disease have a large amount of GM3, since "a fatty acid having 24 carbon atoms, having an OH group as a substituent, and having a double bond" was bonded to (existed in) GM3 particularly, [hC24:1]GM3 was particularly suitable as a marker with regard to specific diseases.

The results of Fig. 5 showed the reasons or mechanisms that the GM3 represented by the formula (1) such as [hC24:1]GM3 was suitable for being used in a method for detecting specific diseases or a method for detecting onset risk of the specific diseases.

### Example 8

### <Comparison of amount of hydroxylated GM3 in each disease>

Each amount of hydroxylated GM3 ("d18:1-h24:0", "d18:1-h24:1", "d18:1-h22:0") and non-hydroxylated GM3 ("d18:1-24:0", "d18:1-24:1", "d18:1-22:0") of (1) normal subjects (n=26), (2) patients with only VFA (n=39), (3) patients with VFA and hyperglycemia (n=15), (4) patients with VFA and dyslipidemia (n=28), and (5) patients with VFA, hyperglycemia and dyslipidemia (namely, patients with metabolic syndrome) (n=17) was measured. The results are shown in Fig. 6. In the figure, "*" shows P≤0.01.

The vertical axis of Fig. 6A shows the total amount (ng/µL) of three kinds of hydroxylated GM3 ("d18:1-h24:0", "d18:1-h24:1", "d18:1-h22:0"), and the vertical axis of Fig. 6B shows the total amount (ng/µL) of three kinds of non-hydroxylated GM3 ("d18:1-24:0", "d18:1-24:1", "d18:1-22:0") .

The results of Fig. 6A showed that the total amount of hydroxylated GM3 ("d18:1-h24:0", "d18:1-h24:1", "d18:1-h22:0") of (5) patients with VFA, hyperglycemia and dyslipidemia was statistically significant compared with that of normal subjects.

On the other hand, the results of Fig. 6B showed that the total amount of non-hydroxylated GM3 ("dl8:1-24:0", "d18:1-24:1", "d18:1-22:0") of any patient (2) to (5) above was not statistically significant compared with that of normal subjects.

### Example 9

### <Correlation between hydroxylated GM3 and each disease marker>

Correlation between hydroxylated GM3 or non-hydroxylated GM3, and each disease marker (total cholesterol, LDL-c, autotaxin, IMT, fasting blood glucose, insulin, HOMA-IR, HbA1c) was analyzed using Spearman's rank correlation. The results are shown in Figs 7 and 8.

Fig. 7 shows correlation between the total amount (ng/µL) of three kinds of hydroxylated GM3 ("d18:1-h24:0", "d18:1-h24:1", "d18:1-h22:0") and each disease marker, and Fig. 8 shows correlation between the total amount (ng/µL) of three kinds of non-hydroxylated GM3 ("d18:1-24:0", "d18:1-24:1", "dl8:1-22:0") and each disease marker.

The results of Fig. 7, the total amount of hydroxylated GM3 ("d18:1-h24:0", "d18:1-h24:1", "d18:1-h22:0") was correlated with total cholesterol, LDL-c, autotaxin, IMT, fasting blood glucose, insulin, HOMA-IR and HbA1c, respectively.

On the other hand, the results of Fig. 8 showed that the total amount of non-hydroxylated GM3 ("d18:1-24:0", "d18:1-24:1", "d18:1-22:0") was correlated with total cholesterol, LDL-c and fasting blood glucose, and not correlated with autotaxin, IMT, insulin, HOMA-IR and HbA1c, respectively.

### <Results of Examples 8 and 9>

The results of Examples 8 and 9 showed that GM3 having an OH group as a substituent in the fatty acid of GM3 (hydroxylated GM3) was useful for detecting specific diseases or lifestyle-related diseases such as metabolic syndrome.

### INDUSTRIAL APPLICABILITY

Detection methods of the present invention can detect at least one disease selected from the group consisting of visceral fat accumulation, hyperglycemia, dyslipidemia, hypertension and atherosclerosis, or **a combination of two or more of said diseases** or detect onset risk of these diseases, can grasp the condition of subjects precisely. Thus, the present invention can be used in fields such as diagnostic agents, reagents for research and clinical examinations.

## Claims

1. A method for detecting at least one disease selected from the group consisting of visceral fat accumulation, hyperglycemia, dyslipidemia, hypertension and atherosclerosis or a combination of two or more of diseases or detecting onset risk of these diseases, which comprises
(a) a step of measuring a ganglioside GM3 in a blood sample derived from a subject,
wherein the ganglioside GM3 has a structure represented by the following formula (1): 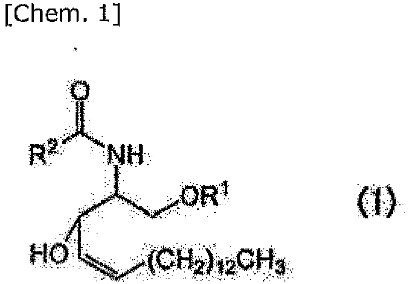 wherein in the formula (1), R¹ represents a glycan constituting the ganglioside GM3, and R²-C(=O)- represents a fatty acid residue having 16 or more and 24 or less carbon atoms which may have a double bond, and having an OH group as a substituent.

2. The detection method according to Claim 1, wherein the disease is metabolic syndrome.

3. The detection method according to Claim 1 or Claim 2, wherein the structure represented by the formula (1) is a hydrocarbon group having 22 or 24 carbon atoms which may have a double bond, and R²-C(=O)- has an OH group as a substituent.

4. The detection method according to Claim 1 or Claim 2, wherein the structure represented by the formula (1) is a hydrocarbon group having 24 carbon atoms which has a double bond, and R²-C(=O)- has an OH group as a substituent.

5. The detection method according to Claim 1 or Claim 2, wherein the structure represented by the formula (1) is a structure represented by the following formula (2): 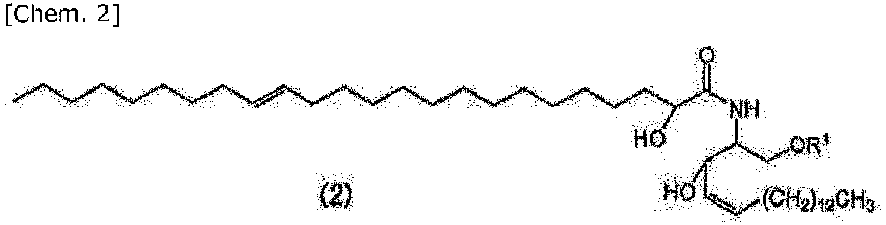 wherein in the formula (2), R¹ represents a glycan constituting the ganglioside GM3.

6. The detection method according to Claim 5, wherein the method further comprises,
(b) a step of comparing an amount of the ganglioside GM3 with the structure represented by the formula (2) measured in the step (a), with an amount of the ganglioside GM3 with the structure represented by the formula (2) in the blood sample derived from a normal subject,
(c) a step of determining the value of a marker with regard to at least one disease selected from the group consisting of visceral fat accumulation, hyperglycemia, dyslipidemia, hypertension and atherosclerosis or a combination of two or more of said diseases of the subject, to be abnormal as compared with those of the normal subject, when there is a significant difference in the step (b), wherein said marker is correlated with said ganglioside GM3.

7. The detection method according to Claim 6, wherein the marker with regard to the visceral fat accumulation, hyperglycemia, dyslipidemia and hypertension is at least one marker selected from the group consisting of autotaxin, HOMA-IR, HbA1c, aspartic acid aminotransferase, alanine-aminotransferase, low-density lipoprotein cholesterol, fasting blood sugar and blood pressure.

8. The detection method according to Claim 6 or Claim 7, wherein the marker with regard to the atherosclerosis is at least one selected from the group consisting of sphingomyelin, lysophosphatidylcholine, C-reactive protein and mean intima-media thickness.

9. The detection method according to any of Claim 1 to Claim 8, wherein the measurement of the ganglioside GM3 is carried out by a method of reacting an antibody to the GM3, a high performance liquid chromatography method, mass spectrometry or liquid chromatography/mass spectrometry.

## Patentansprüche

1. Verfahren zum Nachweis wenigstens einer Krankheit, die aus der Gruppe ausgewählt ist, die aus Viszeralfettakkumulation, Hyperglykämie, Dyslipidämie, Bluthochdruck und Atherosklerose oder einer Kombination von zweien oder mehreren der Krankheiten besteht, oder zum Nachweis des Eintrittsrisikos dieser Krankheiten, umfassend
(a) einen Schritt des Messens eines Gangliosids GM3 in einer von einem Probanden stammenden Blutprobe;
wobei das Gangliosid GM3 eine Struktur aufweist, die durch die folgende Formel (1) dargestellt wird: 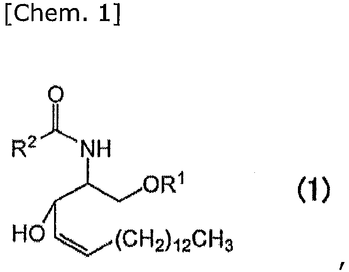 wobei in Formel (1) R¹ für ein Glycan steht, aus dem das Gangliosid GM3 aufgebaut ist, und R²-C(=O)- für einen Fettsäurerest steht, der 16 oder mehr und 24 oder weniger Kohlenstoffatome aufweist, die eine Doppelbindung aufweisen können, und eine OH-Gruppe als Substituent aufweist.

2. Nachweisverfahren gemäß Anspruch 1, wobei es sich bei der Krankheit um das metabolische Syndrom handelt.

3. Nachweisverfahren gemäß Anspruch 1 oder 2, wobei die durch die Formel (1) dargestellte Struktur eine Kohlenwasserstoffgruppe ist, die 22 oder 24 Kohlenstoffatome aufweist, die eine Doppelbindung aufweisen können, und R²-C(=O)- eine OH-Gruppe als Substituent aufweist.

4. Nachweisverfahren gemäß Anspruch 1 oder 2, wobei die durch die Formel (1) dargestellte Struktur eine Kohlenwasserstoffgruppe ist, die 24 Kohlenstoffatome aufweist, die eine Doppelbindung aufweisen können, und R²-C(=O)- eine OH-Gruppe als Substituent aufweist.

5. Nachweisverfahren gemäß Anspruch 1 oder 2, wobei die durch die Formel (1) dargestellte Struktur eine Struktur ist, die durch die folgende Formel (2) dargestellt wird: 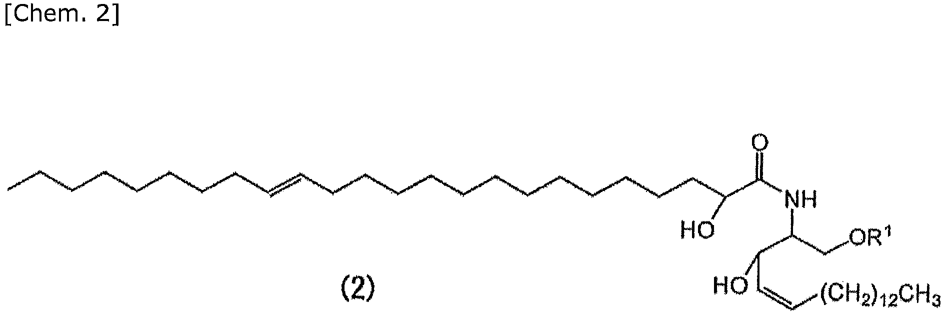 wobei in der Formel (2) R¹ für ein Glycan steht, aus dem das Gangliosid GM3 aufgebaut ist.

6. Nachweisverfahren gemäß Anspruch 5, wobei das Verfahren weiterhin umfasst:
(b) einen Schritt des Vergleichens der in Schritt (a) gemessenen Menge des Gangliosids GM3 mit der durch die folgende Formel (2) dargestellten Struktur mit der Menge des Gangliosids GM3 mit der durch die folgende Formel (2) dargestellten Struktur in einer von einem normalen Probanden stammenden Blutprobe;
(c) einen Schritt des Bestimmens des Werts eines Markers in Bezug auf wenigstens eine Krankheit, die aus der Gruppe ausgewählt ist, die aus Viszeralfettakkumulation, Hyperglykämie, Dyslipidämie, Bluthochdruck und Atherosklerose oder einer Kombination von zweien oder mehreren der Krankheiten besteht, bei dem Probanden als abweichend im Vergleich zu denen des normalen Probanden, wenn es in Schritt (b) einen signifikanten Unterschied gibt, wobei der Marker mit dem Gangliosid GM3 korreliert ist.

7. Nachweisverfahren gemäß Anspruch 6, wobei der Marker in Bezug auf die Viszeralfettakkumulation, Hyperglykämie, Dyslipidämie und den Bluthochdruck wenigstens ein Marker ist, der aus der Gruppe ausgewählt ist, die aus Autotaxin, HOMA-IR, HbA1c, Asparaginsäure-Aminotransferase, Alanin-Aminotransferase, LDL-Cholesterin, Nüchtern-Blutzuckerwert und Blutdruck besteht.

8. Nachweisverfahren gemäß Anspruch 6 oder 7, wobei der Marker in Bezug auf die Atherosklerose wenigstens einer ist, der aus der Gruppe ausgewählt ist, die aus Sphingomyelin, Lysophosphatidylcholin, C-reaktivem Protein und mittlerer Intima-Media-Dicke besteht.

9. Nachweisverfahren gemäß einem der Ansprüche 1 bis 8, wobei die Messung des Gangliosids GM3 mit einem Verfahren, bei dem ein Antikörper gegen das GM3 umgesetzt wird, ein HPLC-Verfahren, Massenspektrometrie oder Flüssigchromatographie/Massenspektrometrie durchgeführt wird.

## Revendications

1. Procédé de détection d'au moins une maladie sélectionnée dans le groupe consistant en l'accumulation de graisse viscérale, l'hyperglycémie, la dyslipidémie, l'hypertension et l'athérosclérose, ou une combinaison de deux maladies ou plus, ou de détection d'un risque de survenue de ces maladies, qui comprend :
(a) une étape de mesure d'un ganglioside GM3 dans un échantillon sanguin provenant d'un sujet,
où le ganglioside GM3 possède une structure représentée par la formule suivante (1) : 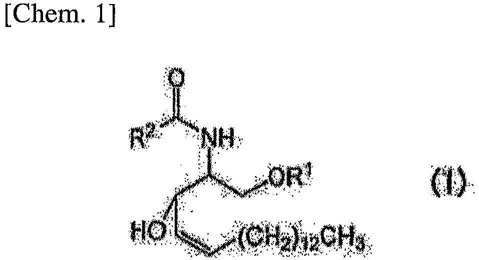 formule (1) dans laquelle R¹ représente un glycane constituant le ganglioside GM3, et R²-C(=O)- représente un résidu acide gras comportant 16 atomes de carbone ou plus et 24 atomes de carbone ou moins, qui peut posséder une double-liaison, et qui présente un groupe OH comme substituant.

2. Procédé de détection selon la revendication 1, dans lequel la maladie est un syndrome métabolique.

3. Procédé de détection selon la revendication 1 ou la revendication 2, dans lequel la structure représentée par la formule (1) est un groupe hydrocarbure comportant 22 ou 24 atomes de carbone et qui peut avoir une double-liaison, et R²-C(=O)- présente un groupe OH comme substituant.

4. Procédé de détection selon la revendication 1 ou la revendication 2, dans lequel la structure représentée par la formule (1) est un groupe hydrocarbure comportant 24 atomes de carbone et ayant une double-liaison, et R²-C(=O)- présente un groupe OH comme substituant.

5. Procédé de détection selon la revendication 1 ou la revendication 2, dans lequel la structure représentée par la formule (1) est une structure représentée par la formule (2) : 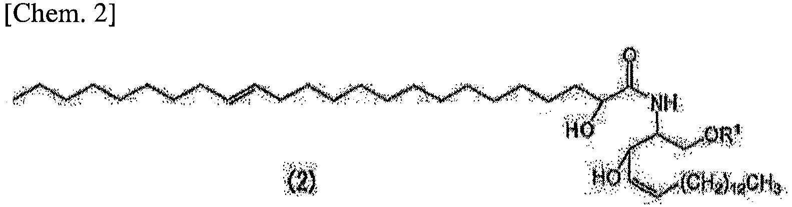 formule (2) dans laquelle R¹ représente un glycane constituant le ganglioside GM3.

6. Procédé de détection selon la revendication 5, dans lequel le procédé comprend en outre
(b) une étape de comparaison d'une quantité du ganglioside GM3 ayant la structure représentée par la formule (2) mesurée à l'étape (a) avec une quantité du ganglioside GM3 ayant la structure représentée par la formule (2) dans l'échantillon sanguin provenant d'un sujet normal,
(c) une étape de détermination de la valeur d'un marqueur concernant au moins une maladie sélectionnée dans le groupe constituant en l'accumulation de graisse viscérale, l'hyperglycémie, la dyslipidémie, l'hypertension et l'athérosclérose, ou une combinaison de deux desdites maladies ou plus du sujet, qui est anormale par rapport à celle d'un sujet normal lorsqu'il existe une différence importante à l'étape (b), ledit marqueur étant corrélé audit ganglioside GM3.

7. Procédé de détection selon la revendication 6, dans lequel le marqueur concernant l'accumulation de graisse viscérale, l'hyperglycémie, la dyslipidémie et l'hypertension est au moins un marqueur sélectionné dans le groupe consistant en l'autotaxine, HOMA-IR, HbA1c, l'acide aspartique aminotransférase, l'alanine-aminotransférase, le cholestérol à lipoprotéine basse densité, la glycémie à jeun et la pression sanguine.

8. Procédé de détection selon la revendication 6 ou la revendication 7, dans lequel le marqueur concernant l'athérosclérose est au moins un marqueur sélectionné dans le groupe consistant en la sphingomyéline, la lysophosphatidylcholine, la protéine C-réactive et l'épaisseur moyenne de l'intima-media.

9. Procédé de détection selon l'une des revendications 1 à 8, dans lequel la mesure du ganglioside GM3 est réalisée par une méthode faisant réagir un anticorps contre le GM3, une méthode de chromatographie liquide haute performance, une spectrométrie de masse ou une chromatographie liquide/spectrométrie de masse.
